# EUROPÄISCHE PATENTSCHRIFT

(11) **EP 0 725 784 B1**
(45) Veröffentlichungstag und Bekanntmachung des Hinweises auf die Patenterteilung: **27.08.1997**
(21) Anmeldenummer: 94931541.0
(22) Anmeldetag: 22.10.1994
(51) Int. Cl.: C07D 493/04, C07D 313/12, A61K 31/335

(54) **DIBENZ[b,e]OXEPINDERIVATE, VERFAHREN ZU IHRER HERSTELLUNG UND DIESE ENTHALTENDE ARZNEIMITTEL**
DIBENZO[b,e]OXEPINE DERIVATIVES, PROCESS FOR PRODUCING THEM AND MEDICAMENTS CONTAINING THEM
DERIVES DE LA DIBENZ[b,e]OXEPINE, PROCEDES POUR LEUR PREPARATION ET MEDICAMENTS CONTENANT CES DERIVES

(30) Priorität: 26.10.1993 DE 4336491
(43) Veröffentlichungstag der Anmeldung: 14.08.1996
(73) Patentinhaber: BOEHRINGER MANNHEIM GMBH, 68298 Mannheim (DE)
(72) Erfinder: FRIEBE, Walter-Gunar, D-68165 Mannheim (DE); SCHEUER, Werner, D-83646 Bad Tölz (DE); TIBES, Ulrich, D-60599 Frankfurt (DE)
(86) Internationale Anmeldenummer: EP9403479
(87) Internationale Veröffentlichungsnummer: WO9511906

(56) Entgegenhaltungen:
- EP-A- 0 436 025
- CHEMICAL ABSTRACTS, vol. 104, no. 23, 1986, Columbus, Ohio, US; abstract no. 202245k, G.MATOLCSY ET AL. 'MOLECULAR MODIFICATIONS OF BENZYLPHENOL AND BENZYL-1,3-BENZODIOXOLE TYPE OF INSECT CHEMOSTERILANTS.' Seite 296 ;Spalte 2 ; & PESTIC. SCI., Bd.17, Nr.1, 1986, BUDAPEST Seiten 13 - 24
- CHEMICAL ABSTRACTS, vol. 104, no. 23, 1986, Columbus, Ohio, US; abstract no. 202245k, G.MATOLCSY ET AL. 'MOLECULAR MODIFICATIONS OF BENZYLPHENOL AND BENZYL-1,3-BENZODIOXOLE TYPE OF INSECT CHEMOSTERILANTS.' Seite 296 ;Spalte 2; & PESTIC. SCI., vol. 17, no. 1, 1986, BUDAPEST pages 13 - 24

## Beschreibung

EP,A,0 436 025 offenbart Dibenz[b,e]oxepinderivate mit antiphlogistischen, entzündungshemmenden und analgetischen Wirkungen. Gegenstand der vorliegenden Erfindung sind neue Dibenz[b,e]oxepinderivate, Verfahren zu deren Herstellung und Arzneimittel, die diese Verbindungen enthalten.

Die Erfindung betrifft Dibenz[b,e]oxepinderivate der allgemeinen Formel I 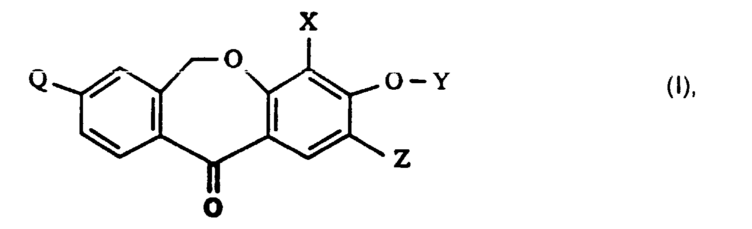 in welcher
- Q: Wasserstoff, eine Hydroxygruppe oder eine C₁- bis C₈-Alkoxygruppe, die gewünschtenfalls mit Carboxyl oder C₁- bis C₆-Alkoxycarbonyl substituiert sein kann, bedeutet und entweder
- Y und Z: gemeinsam eine Gruppe 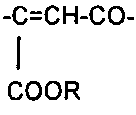 bilden und
- X: für Wasserstoff, C₁- bis C₆-Alkyl oder C₂- bis C₆-Alkenyl steht oder
- Y und X: gemeinsam eine Gruppe 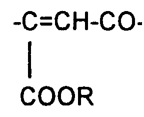 oder eine Alkylenrest von 2 - 4 Kohlenstoffatomen bilden und
- Z: für Wasserstoff, C₁- bis C₆-Alkyl oder C₁- bis C₆-Alkanoyl steht, wobei
- R: Wasserstoff oder einen C₁ - bis C₆-Alkylrest bedeutet,
sowie deren physiologisch verträgliche Salze.

Die Verbindungen der Formel I weisen wertvolle pharmakologische Eigenschaften auf, insbesondere können sie die Aktivität von Phospholipasen hemmen. Sie eignen sich daher zur Behandlung akuter und chronischer, allergischer, nichtallergischer und traumatischer und entzündlicher Erkrankungen, wie beispielsweise rheumatische Arthritis, Osteoarthritis, ulcerative Colitis, akute Pankreatitis, Kontaktdermatitis, entzündliche und allergische Atemwegserkrankungen, septischer Schock, allergischer Schock, Serumkrankheit, Autoimmunerkrankungen, graft-versus-host-Reaktionen, host-versus-graft-Erkrankungen, ischämische oder thrombotische Erkrankungen, beispielsweise Coronarinfarkt oder Cerebralinfarkt.

Alkylreste in den genannten Gruppen Alkyl, Alkoxy und Alkanoyl können geradkettig oder verzweigt sein. Bevorzugte Reste sind der Methyl-, Ethyl-, Propyl-, Isopropyl-, n-Butyl-, Isobutyl-, tert.-Butyl-, n-Pentyl- und 3-Pentylrest.

Alkylenreste können geradkettig oder verzweigt sein. Bevorzugte Reste sind, 1,2-Ethylen, 1,3-Propylen, 1,2-Propylen und 1,4-Butylen.

Außer den in den Beispielen genannten Verbindungen sind Gegenstand der Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise eine Verbindung der allgemeinen Formel II 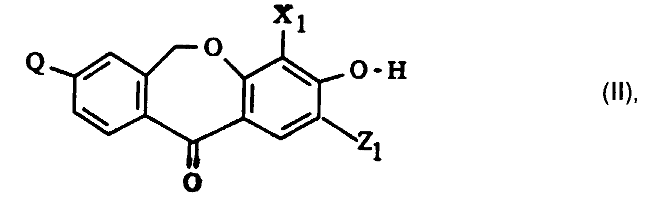 in welcher Q die obengenannte Bedeutung hat und einer der beiden Reste X₁ und Z₁ Acetyl bedeutet, während der andere für Wasserstoff, C₁- bis C₆-Alkyl oder C₂-bis C₆-Alkenyl steht, mit einem Oxalsäurederivat wie beispielsweise Diethyloxalat unter Einwirkung einer Base wie beispielsweise einem Alkalialkoholat umsetzt und cyclisiert oder
eine Verbindung der allgemeinen Formel III 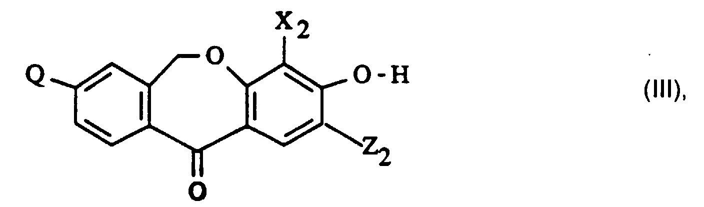 in welcher Q die obengenannte Bedeutung hat und einer der beiden Reste X₂ und Z₂ Wasserstoff bedeutet, während der andere für Wasserstoff, C₁- bis C₆-Alkyl oder C₂- bis C₆-Alkenyl steht, mit einem Halogenfumarsäurederivat wie beispielsweise Bromfumarsäuredimethylester unter Einwirkung einer Base wie beispielsweise einem Alkalialkoholat umsetzt und cyclisiert und anschließend gewünschtenfalls einen oder mehrere der Reste Q, R, X und Z in einen anderen, durch die Definition gegebenen Rest umwandelt und/oder in ein physiologisch verträgliches Salz überführt.

Die Umsetzung von Verbindungen der Formeln II und III erfolgt zweckmäßig in basischem Medium, beispielsweise einem niederen Alkohol wie Methanol, Ethanol oder Isopropanol in Gegenwart eines Alkalialkoholats. Die anschließende Cyclisierung wird beispielsweise in Gegenwart einer Säure wie Schwefelsäure, Polyphosphorsäure, Polyphosphorsäureester oder alkoholische Chlorwasserstoffsäure durchgeführt. Für den Fall, daß Y und X gemeinsam einen Alkylenrest bilden, lassen sich die geeigneten Verbindungen der Formel II ohne Einwirkung eines Oxalsäurederivats säurekatalysiert direkt in die entsprechenden Verbindungen der Formel I überführen.

Eine Umwandlung eines Restes Q in einen anderen, durch den Anspruch definierten Rest Q erfolgt beispielsweise durch Umsetzung einer Verbindungen der Formel I, in der Q für Hydroxyl steht, mit einem Alkylierungsmittel wie beispielsweise einem gewünschtenfalls substituierten Alkylhalogenid.

Eine für Q stehende Alkoxygruppe kann gewünschtenfalls in eine Hydroxygruppe überführt werden, beispielsweise durch Spaltung einer Methoxygruppe mit Bortribromid.

Durch Verseifung einer in Q oder Y und Z oder Y und X enthaltenen Alkoxycarbonylgruppe kann eine Carboxylgruppe erzeugt werden, durch Veresterung einer Carboxylgruppe eine Alkoxycarbonylgruppe.

Steht für X ein Alkenylrest, so kann dieser gewünschtenfalls durch Hydrierung in einen Alkylrest überführt werden.

Die Ausgangsverbindungen II und III sind literaturbekannte Substanzen oder können in Analogie zu literaturbekannten Verfahren hergestellt werden.

Als pharmakologisch verträgliche Salze kommen insbesondere Alkali-, Erdalkali- und Ammoniumsalze sowie gegebenenfalls Salze mit nichttoxischen anorganischen oder organischen Säuren, wie z. B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bemsteinsäure oder Diaminocapronsäure in Frage.

Die Salze erhält man in üblicher Weise z. B. durch Neutralisation der Verbindungen der Formel I mit den entsprechenden Laugen oder Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel. Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke. Lactose, Mannit, Methylcellulose, Talkum, hochdisperse Kieselsäure. höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z. B. mit pulverförmigen, physiologisch verträglichen Verdünnungsmitteln bzw. üblichen Salbengrundlagen vermischt. Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der Erfindung die folgenden Verbindungen bevorzugt:
1. 2-Carboxymethoxy-7. 13-dihydro-5H- 10,12-dioxa-benzo[4,5]cyclohepta[1,2-b]naphthalin-5,7-dion-9-carbonsäure-dinatrium
2. 7,12-Dihydro-5-propyl-1H-4, 13-dioxabenzo[4,5]cyclohepta[1,2-a]naphthalin-1,7-dion-3-carbonsäureethylester
3. 11-Allyl-7, 13-dihydro-2-hydroxy-5H-10,12-dioxa-benzo[4,5]cydohepta[1,2-b]naphthalin-5,7-dion-9-carbonsäureethylester
4. 11-Allyl-7,13-dihydro-2-ethoxycarbonylmethoxy-5H-10,12-dioxabenzo[4,5]cyclohepta[1,2-b]naphthalin-5,7-dion-9-carbonsäureethylester
5. 11-Allyl-7, 13-dihydro-2-octyloxy-5H-10,12-dioxa-benzo[4,5]cydohepta[1,2-b]naphtahlin-5,7-dion-9-carbonsäureethylester

### Beispiel 1

### 7,12-Dihydro-1H-4,13-dioxa-benzo[4,5]cyclohepta[1,2-a]naphthalin-1,7-dion-3-carbonsäureethylester

Zu einer Lösung von 4.14 g (180 mmol) Natrium in 250 ml Ethanol fügt man 8.04 g (30 mmol)4-Acetyl-6, 11-dihydro-3-hydroxy-dibenz[b,e]oxepin-11-on, erwärmt 10 min zum Rückfluß, kühlt auf Raumtemperatur, versetzt mit 14.6 g (100 mmol) Diethyloxalat, erwärmt 90 min zum Rückfluß, läßt abkühlen und filtriert. Der Niederschlag wir in 100 ml Ethanol aufgenommen, die Suspension mit Chlorwasserstoff gesättigt und anschließend 1 h auf 50°C erwärmt. Nach Abkühlung gießt man auf Eis und filtriert. Es verbleiben 7.8 g Titelverbindung (74 % d. Th.) vom Schmp. 178-180°C (aus Ethanol).

### Beispiel 2

In analoger Weise wie in Beispiel 1 beschrieben erhält man:

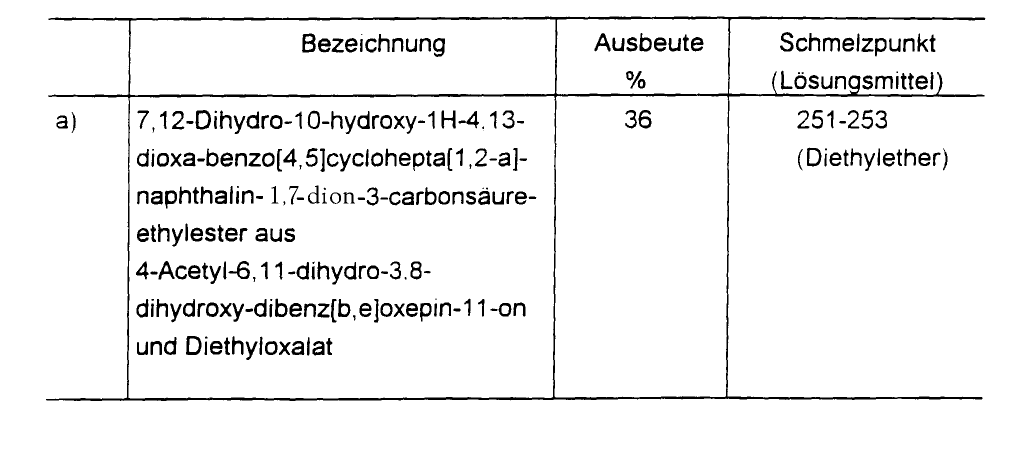

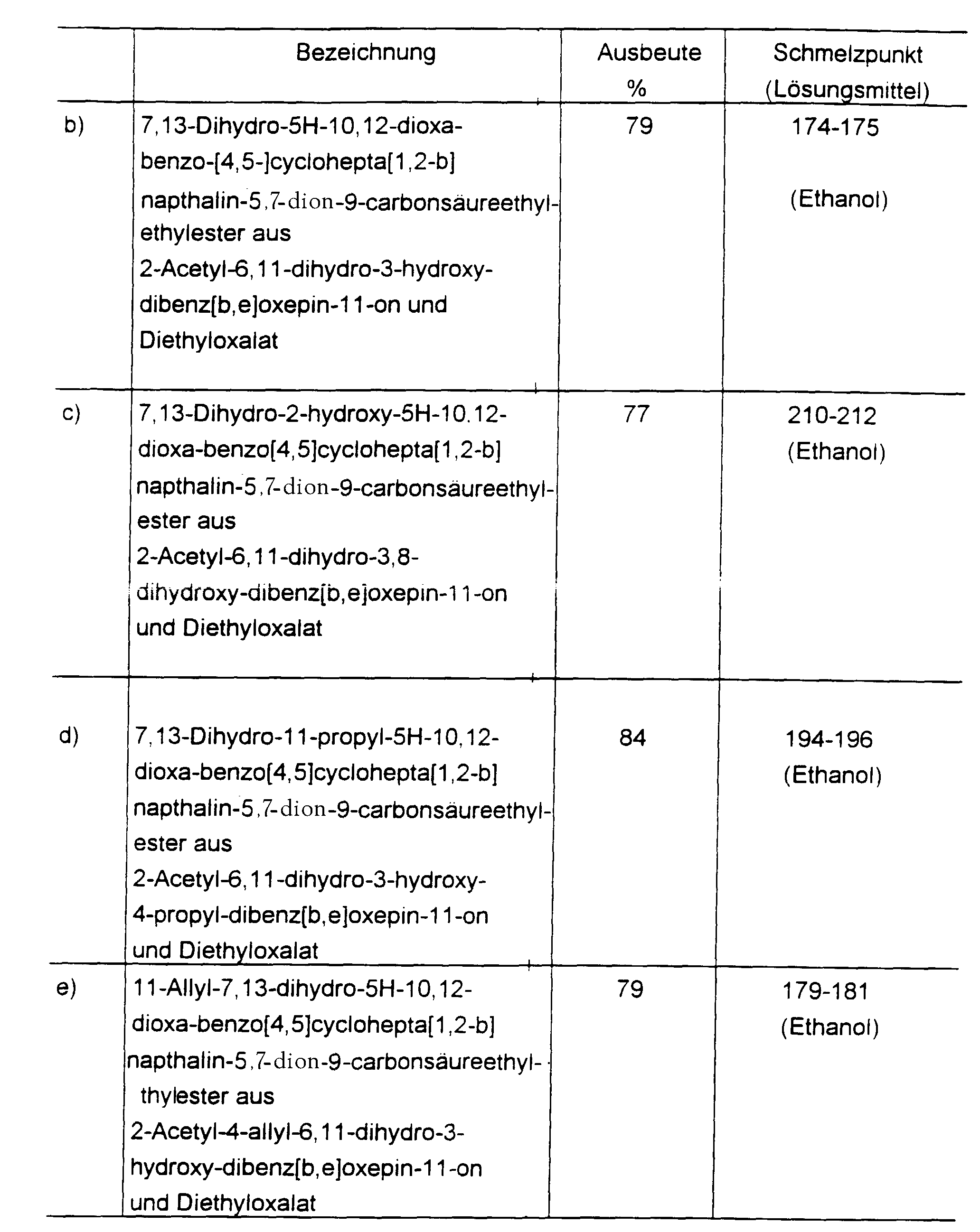

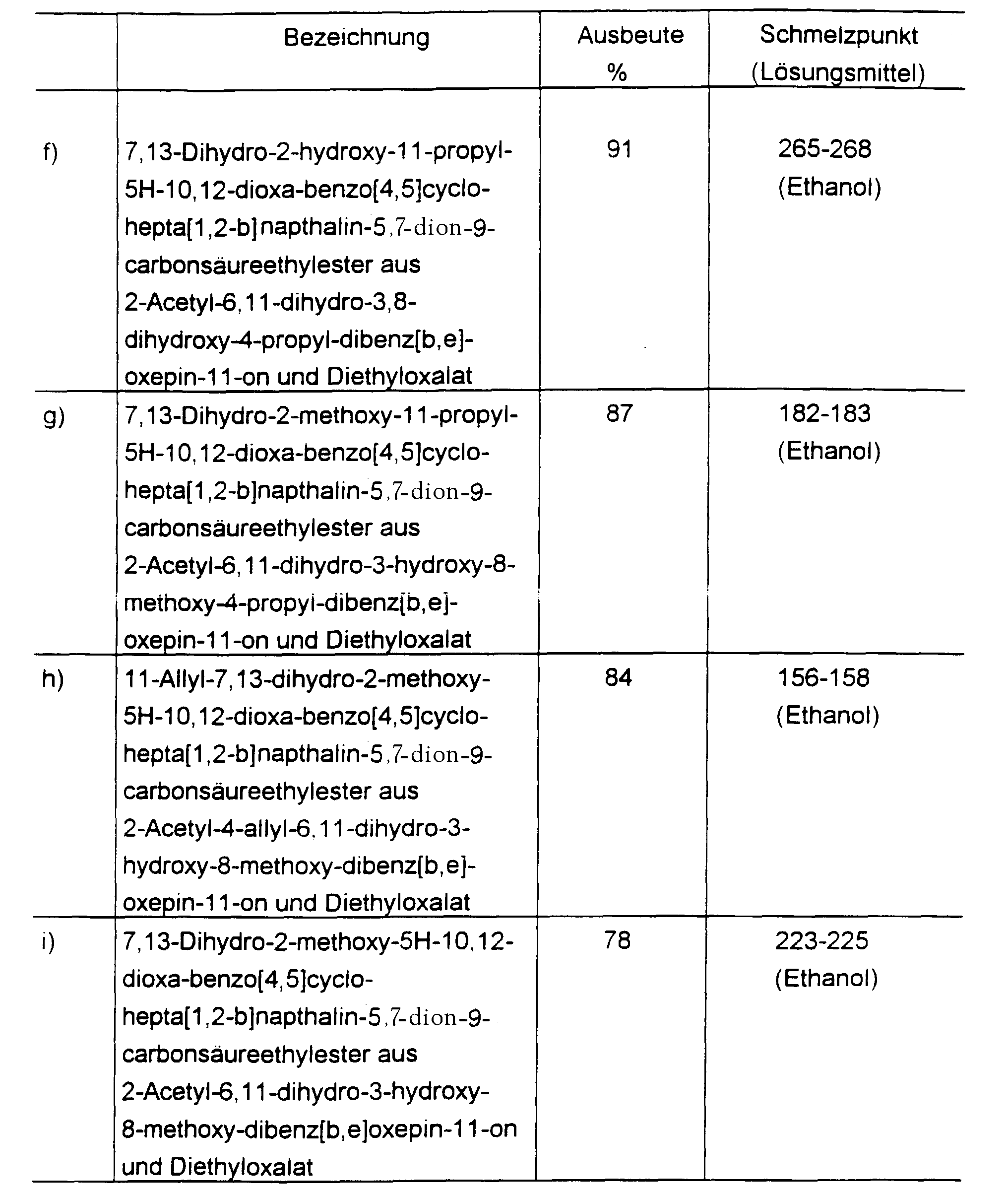

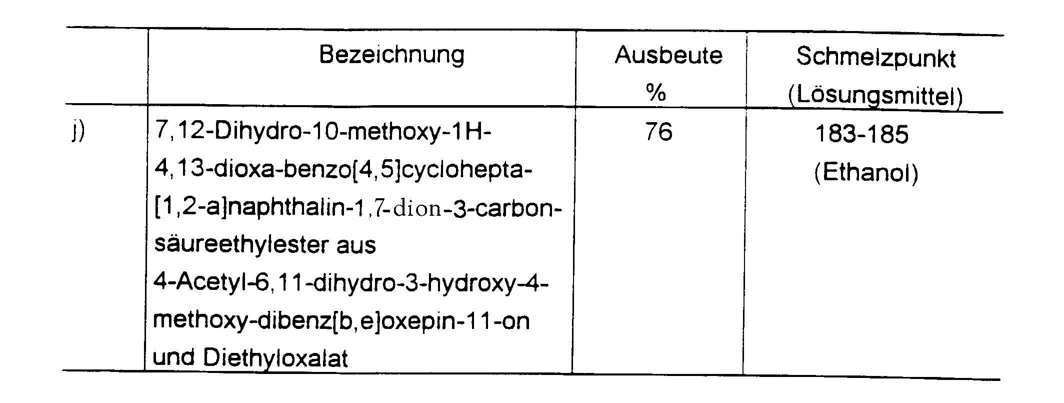

### Beispiel 3

### 7,13-Dihydro-2-octyloxy-5H-10,12-dioxa-benzo[4,5]cyclohepta[1,2-b]naphthalin-5,7-dion-9-carbonsäureethylester

Eine Mischung aus 3.7 g (10 mmol) Verbindung des Beispiels 2 c, 50 ml Dimethylformamid, 1.4 g Kaliumcarbonat und 1.75 ml 1-Bromoctan wird 6 h auf 80°C erwärmt, anschließend filtriert und das Filtrat eingeengt. Man nimmt in Ethylacetat auf und chromatographiert an Kieselgel (Elutionsmittel Ethylacetat : Isohexan 1:1). Nach Verreiben mit Ether verbleiben 2.4 g Titelverbindung (50 % d. Th.) vom Schmp. 103-105°C.

### Beispiel 4

In analoger Weise wie in Beispiel 3 beschrieben erhält man:

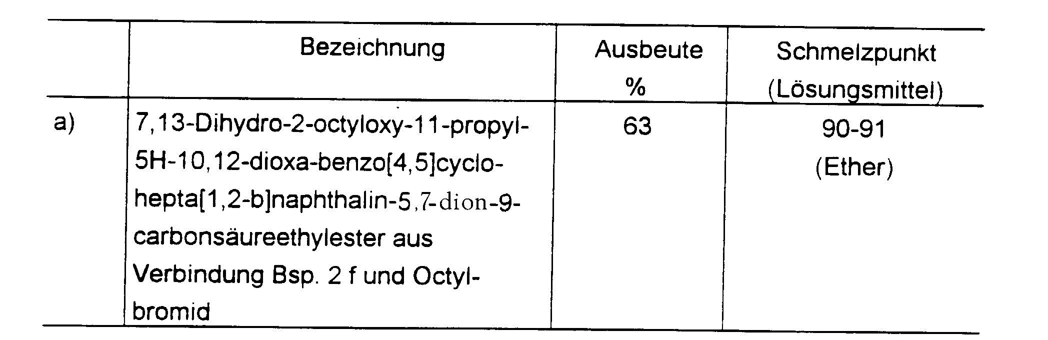

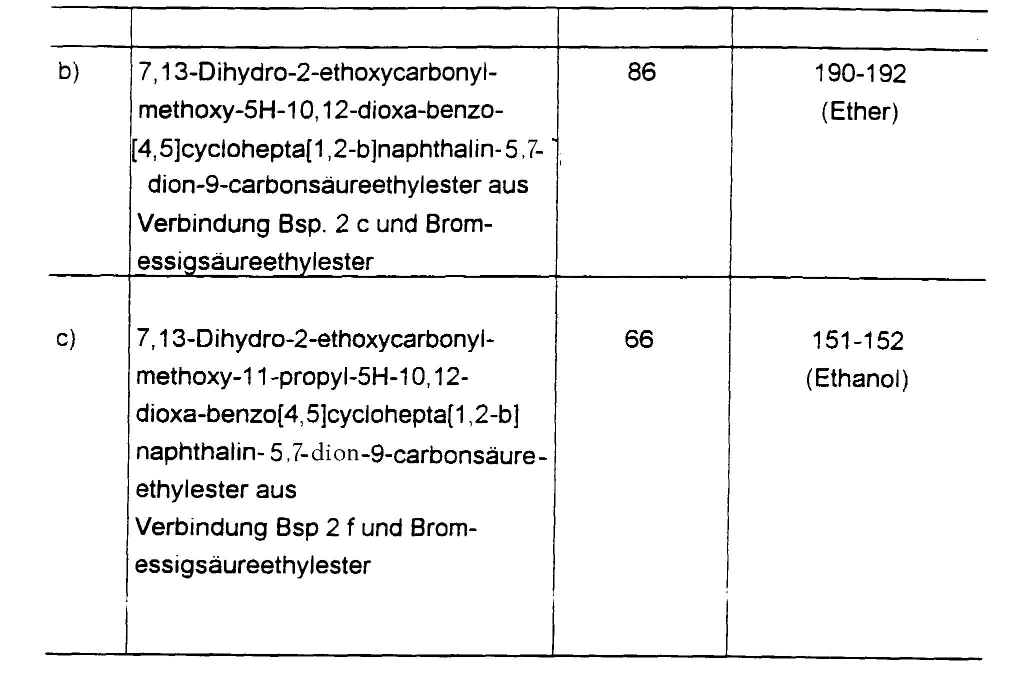

### Beispiel 5

### 7,13-Dihydro-5H-10,12-dioxa-benzo[4,5]cyclohepta[1,2-b]naphthalin-5,7-dion-9-carbonsäure-natrium

Zu einer Lösung von 6.65 g (19 mmol) Verbindung des Beispiels 2 b in 150 ml Ethanol gibt man 19 ml N Natronlauge und 30 ml Wasser. erwärmt 30 min auf 70°C, läßt abkühlen und filtriert den Niederschlag ab. Man erhält 5.2 g Titelverbindung (80 % d. Th.) vom Zersetzungspunkt oberhalb 290°C.

### Beispiel 6

In analoger Weise wie in Beispiel 5 beschrieben erhält man:

| | Bezeichnung | Ausbeute % | Schmelzpunkt (Lösungsmittel) |
|---|---|---|---|
| a) | 7,13-Dihydro-2-hydroxy-5H-10,12-dioxa-benzo[4,5]cyclohepta[1,2-b]naphthalin-5,7-dion-9-carbonsäure-natrium aus Verb. Bsp. 2 c | 67 | über 280 (Wasser) |
| b) | 7,13-Dihydro-2-octyloxy-5H-10,12-dioxa-benzo[4,5]cyclohepta[1,2-b]naphthalin-5,7-dion-9-carbonsäure-natrium aus Verb. Bsp. 3 | 88 | über 280 (Wasser) |
| c) | 7,13-Dihydro-2-ethoxycarbonylmethoxy-5H-10,12-dioxabenzo[4,5]cyclohepta[1,2-b]naphthalin-5,7-dion-9-carbonsäure-natrium aus Verb. Bsp. 4 b | 56 | über 280 (Wasser) |
| d) | 7,13-Dihydro-2-methoxy-5H-10,12-dioxa-benzo[4,5]cyclohepta[1,2-b]naphthalin-5,7-dion-9-carbonsäure-natrium aus Verb. Bsp. 2 i | 47 | über 280 (Wasser) |
| e) | 7,12-Dihydro-1H-4,13-dioxa-benzo[4,5]cyclohepta[1,2-a]naphthalin-1,7-dion-3-carbonsäure-natrium aus Verb. Bsp. 1 | 75 | über 280 (Wasser) |
| f) | 7,12-Dihydro-10-methoxy-1H-4,13-dioxa-benzo-[4,5]cyclohepta[1,2-a]naphthalin-1,7-dion-3-carbonsäure-natrium aus Verb. Bsp. 2 j | 52 | über 280 (Wasser) |

### Beispiel 7

### 4-Acetyl-9-methoxy-2-methyl-1,2,6,11-tetrahydro-3,12-dioxabenzo[4,5]cyclohepta[1,2-e]inden-6-on

Zu einer Mischung aus 9.2. g Phosphorpentoxid und 6 ml Ethanol gibt man bei 100°C 3.9 g (11 mmol) 2-Acetyl-4-allyl-6,11-dihydro-3-hydroxy-8-methoxydibenz[b,e]oxepin-11-on, erhitzt 30 min auf 140°C, gießt auf Eis und filtriert. Nach Chromatographie an Kieselgel (Elutionsmittel: Ethylacetat : Isohexan 1:1) isoliert man 0.9 g Titelverbindung (23 % d. Th.) vom Schmp. 159 - 160°C (aus Ether).

Als Nebenprodukt kann 5-Acetyl-7,12-dihydro-10-methoxy-1H-4,13-dioxabenzo[4,5]cyclohepta[1,2-a]napthalin-7-on nachgewiesen werden.

### Beispiel 8

### Hemmung der PLA₂-Aktivität

Als typischer Repräsentant einer PLA₂ wurde die humane rekombinante Typ-II-PLA₂ (= synoviale PLA₂) zur Testung eingesetzt.

Die Tabelle enthält die prozentuale in-vitro Hemmung dieses Enzyms durch die beispielhaften Verbindungen der Beispiele 2 a), 2 c) und 4 a). Das Enzym wurde dosisabhängig gehemmt, wobei mit 10µg/ml schon 78,61 und 49 % Hemmung erreicht wurde.

Demgegenüber sieht man in der Tabelle, daß Indometacin das Enzym zwar auch hemmte, jedoch nur mit maximal 52 % bei der höchsten Konzentration von 100 µg/ml. Hierin dokumentiert sich die Überlegenheit der neuen sPLA2-lnhibitoren gegenüber einem Cyclooxygenasehemmer.

**Tabelle:**

| Hemmung der sPLA₂-Enzymaktivität durch tetracyclische Dibenzoxepinderivate und Indometacin | | | | |
|---|---|---|---|---|
| | **Substanzen** | | | |
| **Konzentrationen** | **Bsp. 2 c)** | **Bsp. 2 a)** | **Bsp. 4 a)** | **Indometacin** |
| 100 µg/ml | n.b. | n.b. | n.b. | 52 |
| 10 µg/ml | 78 | 61 | 49 | 10 |
| 1 µg/ml | 73 | 52 | 17 | 0 |
| 0,1 µg/ml | 12 | 28 | 16 | n.b. |
| Mittelwerte aus 3 Versuchen (Doppelbestimmungen), n.b. = nicht bestimmt | | | | |

## Patentansprüche

1. Dibenz[b,e]oxepin-Derivate der Formel I 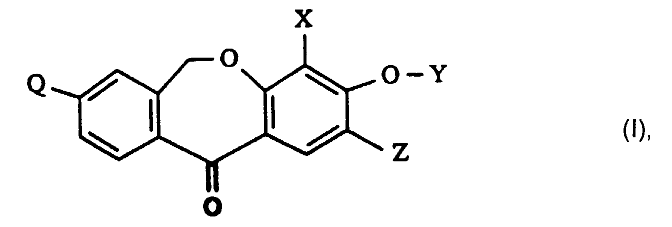 in welcher
Q Wasserstoff, eine Hydroxygruppe oder eine C₁ - bis C₈-Alkoxygruppe, die gewünschtenfalls mit Carboxyl oder C₁- bis C₆-Alkoxycarbonyl substituiert sein kann, bedeutet und entweder
Y und Z gemeinsam eine Gruppe 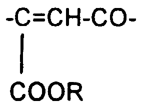 bilden und
X für Wasserstoff, C₁- bis C₆-Alkyl oder C₂- bis C₆-Alkenyl steht oder
Y und X gemeinsam eine Gruppe 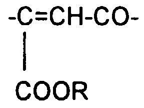 oder eine Alkylenrest von 2 - 4 Kohlenstoffatomen bilden und
Z für Wasserstoff, C₁- bis C₆-Alkyl oder C₁- bis C₆-Alkanoyl steht, wobei
R Wasserstoff oder einen C₁- bis C₆-Alkylrest bedeutet,
sowie deren physiologisch verträgliche Salze.

2. Verfahren zur Herstellung von Dibenz[b,e]oxepin-Derivaten der Formel I, 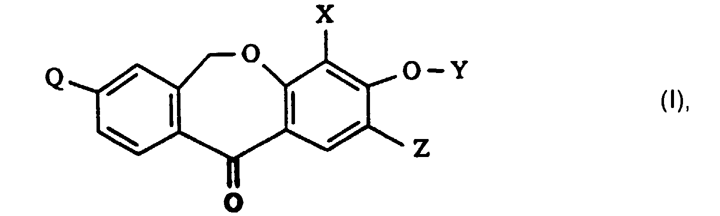 in welcher
Q Wasserstoff, eine Hydroxygruppe oder eine C₁ - bis C₈-Alkoxygruppe, die gewünschtenfalls mit Carboxyl oder C₁- bis C₆-Alkoxycarbonyl substituiert sein kann, bedeutet und entweder
Y und Z gemeinsam eine Gruppe 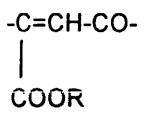 bilden und
X für Wasserstoff, C₁- bis C₆-Alkyl oder C₂- bis C₆-Alkenyl steht oder
Y und X gemeinsam eine Gruppe 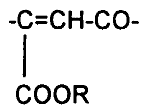 oder eine Alkylenrest von 2 - 4 Kohlenstoffatomen bilden und
Z für Wasserstoff, C₁- bis C₆-Alkyl oder C₁- bis C₆-Alkanoyl steht, wobei
R Wasserstoff oder einen C₁- bis C₆-Alkylrest bedeutet,
sowie deren physiologisch verträgliche Salze,
dadurch gekennzeichnet, daß man in an sich bekannter Weise
a) eine Verbindung der Formel II 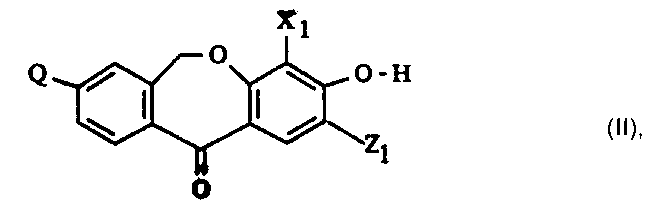 in welcher Q die oben genannte Bedeutung hat und einer der beiden Reste X₁ und Z₁ Acetyl bedeutet, während der andere für Wasserstoff, C₁- bis C₆-Alkyl oder C₂-bis C₆-Alkenyl steht, mit einem Oxalsäurederivat wie beispielsweise Diethyloxalat unter Einwirkung einer Base wie beispielsweise einem Alkalialkoholat umsetzt und cyclisiert
oder
b) eine Verbindung der Formel III 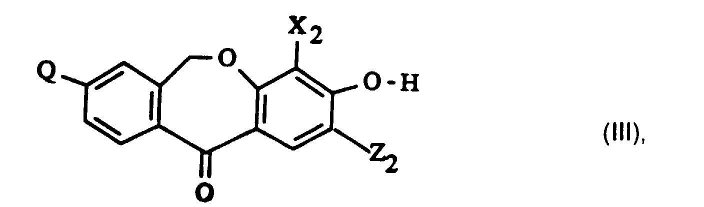 in welcher Q die oben genannte Bedeutung hat und einer der beiden Reste X₂ und Z₂ Wasserstoff bedeutet, während der andere für Wasserstoff, C₁- bis C₆-Alkyl oder C₂- bis C₆-Alkenyl steht, mit einem Halogenfumarsäurederivat wie beispielsweise Bromfumarsäuredimethylester unter Einwirkung einer Base wie beispielsweise einem Alkalialkoholat umsetzt und cyclisiert
und anschließend gewünschtenfalls einen oder mehrere der Reste Q, R, X und Z in einen anderen, durch die Definition gegebenen Rest umwandelt und/oder in ein physiologisch verträgliches Salz überführt.

3. Arzneimittel, enthaltend mindestens eine Verbindung gemäß Anspruch 1 neben üblichen Träger- und Hilfsstoffen.

4. Verwendung von Verbindungen gemäß Anspruch 1 zur Herstellung von Arzneimitteln zur Behandlung akuter und chronischer, allergischer, nichtallergischer und traumatischer und entzündlicher Erkrankungen.

## Claims

1. Dibenz[b,e]oxepin derivatives of formula I 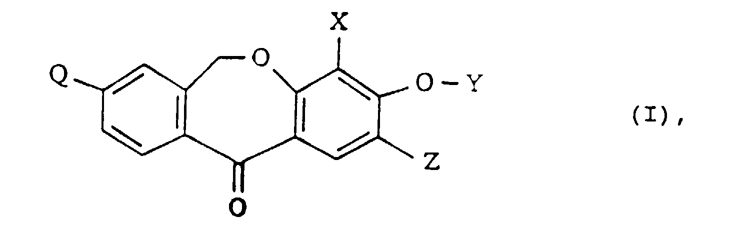 in which
Q denotes hydrogen, a hydroxy group or a C₁ to C₈ alkoxy group which can be substituted if desired, by carboxyl or C₁ to C₆ alkoxycarbonyl and either
Y and Z together form a group 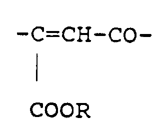 and
X represents hydrogen, C₁ to C₆ alkyl or C₂ to C₆ alkenyl or
Y and X together form a group 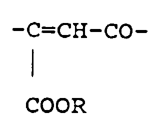 or an alkylene residue of 2 - 4 carbon atoms and
Z represents hydrogen, C₁ to C₆ alkyl or C₁ to C₆ alkanoyl, wherein
R denotes hydrogen or a C₁ to C₆ alkyl residue
as well as physiologically tolerated salts thereof.

2. Process for the production of dibenz[b,e]oxepin derivatives of formula I 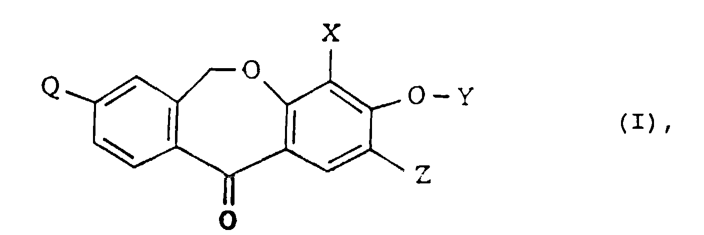 in which
Q denotes hydrogen, a hydroxy group or a C₁ to C₈ alkoxy group which can be substituted if desired, by carboxyl or a C₁ to C₆ alkoxycarbonyl and either
Y and Z together form a group 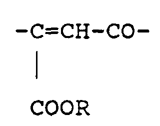 and
X represents hydrogen, C₁ to C₆ alkyl or C₂ to C₆ alkenyl or
Y and X together form a group 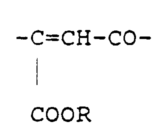 or an alkylene residue of 2 - 4 carbon atoms and
Z represents hydrogen, C₁ to C₆ alkyl or C₁ to C₆ alkanoyl, wherein
R denotes hydrogen or a C₁ to C₆ alkyl residue
as well as physiologically tolerated salts thereof,
wherein in a known manner
a) a compound of formula II 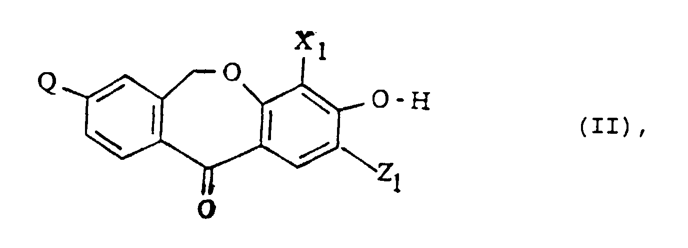 in which Q has the above-mentioned meaning and one of the two residues X₁ and Z₁ denotes acetyl whereas the other represents hydrogen, C₁ to C₆ alkyl or C₂ to C₆ alkenyl, is reacted with an oxalic acid derivative such as for example diethyl oxalate under the influence of a base such as for example an alkali alcoholate and cyclized
or
b) a compound of formula III 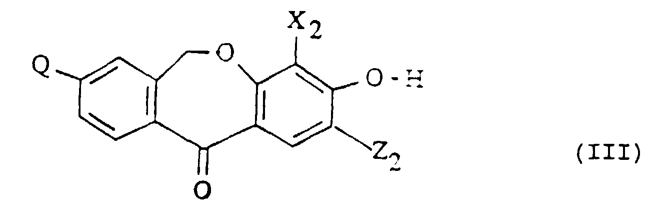 in which Q has the above-mentioned meaning and one of the two residues X₂ and Z₂ denotes hydrogen whereas the other represents hydrogen, C₁ to C₆ alkyl or C₂ to C₆ alkenyl is reacted with a halogenfumaric acid derivative such as for example bromofumaric acid dimethyl ester under the influence of a base such as for example an alkali alcoholate and cyclized
and subsequently, if desired, one or several of the residues Q, R, X and Z are converted into another residue given by the definition and/or converted into a physiologically tolerated salt.

3. Pharmaceutical agent containing at least one compound as claimed in claim 1 in addition to the usual carrier and auxiliary substances.

4. Use of compounds as claimed in claim 1 for the production of pharmaceutical agents for the treatment of acute and chronic, allergic, non-allergic and traumatic and inflammatory diseases.

## Revendications

1. Dérivés de dibenz[b,e]oxépine de formule I 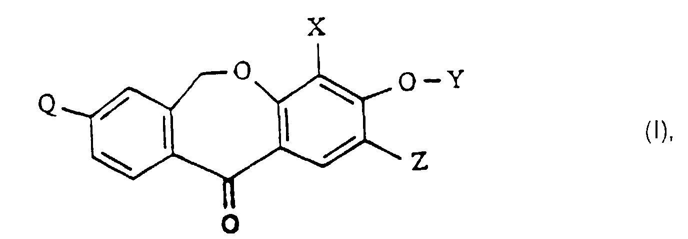 dans laquelle
Q représente un atome d'hydrogène, un groupe hydroxy ou un groupe alcoxy en C₁-C₈, qui peut être éventuellement substitué par un groupe carboxyle ou un groupe alcoxycarbonyle en C₁-C₆, et soit
Y et Z forment ensemble un groupe 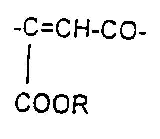 et
X représente un atome d'hydrogène, un groupe alkyle en C₁-C₆ ou alcényle en C₂-C₆, soit
Y et Z forment ensemble un groupe 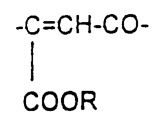 ou un groupe alkylène ayant de 2 à 4 atomes de carbone et
Z représente un atome d'hydrogène, un groupe alkyle en C₁-C₆ ou alcanoyle en C₁-C₆, où
R représente un atome d'hydrogène ou un groupe alkyle en C₁-C₆,
ainsi que leurs sels physiologiquement acceptables.

2. Procédé de préparation de dérivés de dibenz[b,e]oxépine de formule I 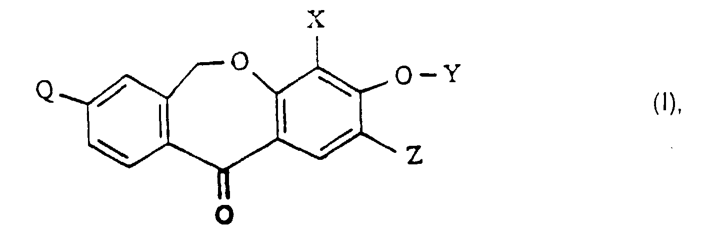 dans laquelle
Q représente un atome d'hydrogène, un groupe hydroxy ou un groupe alcoxy en C₁-C₈, qui peut être éventuellement substitué par un groupe carboxyle ou un groupe alcoxycarbonyle en C₁-C₆, et soit
Y et Z forment ensemble un groupe 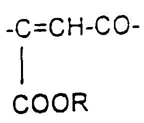 et
X représente un atome d'hydrogène, un groupe alkyle en C₁-C₆ ou alcényle en C₂-C₆, soit
Y et Z forment ensemble un groupe 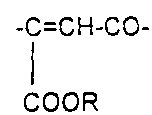 ou un groupe alkylène ayant de 2 à 4 atomes de carbone et
Z représente un atome d'hydrogène, un groupe alkyle en C₁-C₆ ou alcanoyle en C₁-C₆, où
R représente un atome d'hydrogène ou un groupe alkyle en C₁-C₆,
ainsi que leurs sels physiologiquement acceptables,
caractérisé en ce que de manière connue en soi, on fait réagir
a) un composé de formule Il 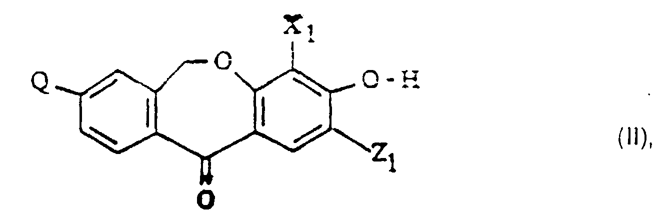 dans laquelle Q a la signification mentionnée ci-dessus et l'un des deux restes X₁ et Z₁ représente un groupe acétyle tandis que l'autre représente un atome d'hydrogène, un groupe alkyle en C₁-C₆ ou alcényle en C₂-C₆, avec un dérivé de l'acide oxalique tel que par exemple l'oxalate de diéthyle, sous l'action d'une base telle que par exemple un alcoolate de métal alcalin, et on effectue une cyclisation, ou
b) un composé de formule III 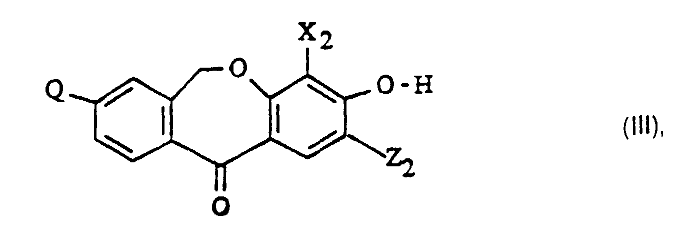 dans laquelle Q a la signification mentionnée ci-dessus et l'un des deux restes Y₂ et Z₂ représente un atome d'hydrogène tandis que l'autre représente un atome d'hydrogène, un groupe alkyle en C₁-C₆ ou alcényle en C₂-C₆, avec un dérivé d'un acide halogénofumarique tel que par exemple l'ester diméthylique de l'acide bromofumarique, sous l'action d'une base telle que par exemple un alcoolate de métal alcalin, et ensuite, on effectue une cyclisation,
et ensuite, on transforme éventuellement un ou plusieurs des restes Q, R, X ou Z en un autre reste répondant à la définition et/ou on le transforme en un de ses sels physiologiquement acceptables.

3. Médicament contenant au moins un composé selon la revendication 1, en plus de matériaux de support et d'adjuvants usuels.

4. Utilisation de composés selon la revendication 1 pour la préparation de médicaments destinés au traitement d'affections aiguës ou chroniques, de type allergique, non-allergique ou traumatique, et des affections de type inflammatoire.
